(19)

Europäisches
Patentamt
European
Patent Office
Office européen
des brevets

(11) **EP 2 008 702 A1**

(12) **EUROPEAN PATENT APPLICATION**

(43) Date of publication:
**31.12.2008 Bulletin 2009/01**

(21) Application number: **07110978.9**

(22) Date of filing: **25.06.2007**

(51) Int Cl.:
**B01D 61/02** (2006.01)     **A23F 3/16** (2006.01)
**A23L 1/305** (2006.01)

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(71) Applicant: **Unilever PLC**
**London**
**Greater London EC4P 4BQ (GB)**

(72) Inventors:
• **Jones, Meirion Gayford**
  **Singleton Park, Sansea SA2 8PP (GB)**
• **Mandale, Stephen John**
  **Singleton Park, Sansea SA2 8PP (GB)**

(74) Representative: **Hugot, Alain**
**Unilever Patent Group**
**Colworth House**
**Sharnbrook**
**Bedford, MK44 1LQ (GB)**

(54) **Membrane filtration process and apparatus**

(57)     The present invention provides a process for purifying a solute from a tea extraction solution as e.g. theanine using membrane filtration whereby enrichment of a solute in a permeate from a first membrane filtration step is achieved by passing the permeate through the same, or at least a substantially identical, nanofiltration membrane filter. An apparatus for performing the process is also described.

EP 2 008 702 A1

**Description**

**TECHNICAL FIELD OF THE INVENTION**

The present invention relates to a purification process employing membrane filtration and an apparatus therefore. In particular, the present invention relates to purifying a target solute from a feed solution comprising impurities by employing a plurality of substantially identical membrane filters.

**BACKGROUND TO THE INVENTION**

Food and beverage products which have functional benefits going beyond simple nutrition are becoming popular. In particular, such products which deliver health benefits and/or enhance physical or mental performance are desired. In general the functional benefits arise owing to the presence of certain bioactive compounds in the products. For example, International Patent Application no. PCT/EP2005/012464 (Unilever PLC et al.) describes consumable compositions which comprise specific levels of theanine and caffeine and which are shown to provide noticeable improvements in concentration, mental focus and/or alertness of an individual consuming the compositions.

Another present trend is for food and beverage products made from only natural ingredients and which are substantially free from additives which are perceived as unnatural and/or artificial. Thus products which comprise bioactive compounds derived from natural sources are particularly valuable.

Unfortunately, many bioactive compounds occur in only small quantities in natural sources. For example, theanine is an amino acid which, within the plant kingdom, is uniquely found in tea (*Camellia sinensis*). Furthermore, theanine only comprises about 2% by weight of the extractable tea solids in tea plant material.

There is therefore a need to provide methods for the concentration of naturally occurring actives without adding synthetic compounds.

Membrane filtration allows for the physical separation and purification of substances and in general does not require use of synthetic compounds. Thus in order to meet the aforementioned need, processes have been developed which use membrane filtration to purify actives from natural sources. For example, a process has been proposed to purify theanine from tea extracts in international patent application no. PCT/EP2005/010376. This application discloses a process to provide a theanine-rich tea extract by performing a cold water extraction of tea plant material and then passing the extract through a nanofiltration step wherein the nanofilter has a theanine rejection of less than 50%. The process results in a permeate having tea solids enriched in theanine.

It has also been known in the art to use a series of membrane filters for the physical separation and purification of substances. In this case, each of the membrane filters will have different characteristics such that substances which pass easily through one filter will tend to be rejected by a later filter. For example, PCT/EP2005/010376 also discloses passing the enriched permeate through another nanofilter, however, this time one with a theanine rejection of greater than 80%. Thus in the first nanofiltration step of PCT/EP2005/010376, the permeate is enriched in theanine, whilst in the second the retentate is enriched in the theanine.

Surprisingly, we have now found that significant further enrichment of a permeate from a first membrane filtration step can be achieved by passing the permeate through the same, or at least a substantially identical, membrane filter.

**DEFINITIONS**

Impurities, Purification and Enrichment

As used herein, the term "impurities" refers to solutes in the feed solution other than the target solute. It does not include the solvent in the feed solution. Thus for an aqueous feed solution the impurities are all of the solids except for the target solute.

Where a given solution is said to be "enriched" in the target solute, it is meant that the weight ratio of the target solute to impurities is increased. Similarly "purification" refers to increasing the weight ratio of the target solute to the impurities.

Rejection Percentage

The rejection percentage ($Q_i$) of the i<sup>th</sup> membrane filter is given by formula (1):

$$Q_i = 100\ ([C_f - C_{p_i}]\ /\ C_f)\ ,\qquad\qquad (1)$$

Wherein $C_f$ is the concentration (% w/w) of the target solute in the feed solution and $Cp_i$ is the concentration (% w/w) of the target solute in the permeate when the feed solution is passed through the filter. Note that $Q_i$ is characterised for each filter using an identical solution of the target solute (the feed solution) at the same trans-membrane pressure (TMP) and temperature. For example if each of the filters was a nanofilter then $Q_i$ would usually be determined by passing the feed solution individually through each filter at a TMP of 5 bar and a temperature of 20°C.

Tea Extract

As used herein, the term "tea extract" refers to dry material from the leaves and/or stem of *Camellia sinensis* var. *sinensis* and/or *Camellia sinensis* var. *assamica.* The leaves and/or stem may have been subjected to a so-called "fermentation" step
wherein they are oxidised by certain endogenous enzymes that are released during the early stages of "black tea" manufacture. This oxidation may even be supplemented by the action of exogenous enzymes such as oxidases, laccases and peroxidases. Alternatively the leaves may have been partially fermented ("oolong" tea) or substantially unfermented ("green tea").

**SUMMARY OF THE INVENTION**

The present invention provides a process for purifying a target solute from a feed solution comprising impurities, the process comprising:

> i) passing the feed solution through a first membrane filtration step thereby to form a first retentate and a first permeate, wherein the first permeate is enriched in the target solute with respect to the feed solution; and then
> ii) passing at least a portion of the first permeate through a second membrane filtration step thereby to form a second retentate and a second permeate, wherein the second permeate is enriched in the target solute with respect to first permeate;

and wherein the membrane filter used in the second membrane filtration step (ii) is substantially identical to the membrane filter used in the first membrane filtration step (i).
When two or more membrane filters are referred to herein as being "substantially identical" it is meant that they have substantially the same rejection behaviour towards the target solute in the feed solution. In particular, it is preferred that a given membrane filter is substantially identical to the filter used in the first filtration step such that the ratio $Q_1:Q_i$ is from 1.2:1 to 1:1.2, where $Q_1$ is the rejection percentage of the membrane filter used in the first filtration step (i) for the target solute and $Q_i$ is the rejection percentage of the given membrane filter for the target solute. More preferably the ratio $Q_1:Q_i$ is from 1.15:1 to 1:1.15, even more preferably from 1.1:1 to 1:1.1 and most preferably from 1.05:1 to 1:1.05.
To minimise the need for pumps and extra filtration equipment, it is preferred that the same membrane filter is used in both filtration steps (i) and (ii). In fact any or all additional filtration steps may use the same membrane filter as used in step (i).
Surprisingly, we have found that significant further enrichment of the permeate from the second membrane filtration step (ii) can be achieved by passing the second permeate through the same, or at least a substantially identical, membrane filter. Thus in a preferred embodiment at least a portion of the second permeate is passed through at least one further membrane filtration step using a membrane filter substantially identical to the membrane filter used in the first membrane filtration step (i). More preferably at least a portion of the second permeate is passed through a series of further membrane filtration steps, each further membrane filtration step using a membrane filter substantially identical to the membrane filter used in the first membrane filtration step (i). For example, there may be a series of 2 to 5 further membrane filtration steps, more preferably 2 to 3.
There is no limitation to the membrane filters used in the present invention. For example, the membrane filter used in the first membrane filtration step may be selected from a microfilter, an ultrafilter, a nanofilter and a reverse-osmosis filter. We have found that particularly efficient results are obtained when the filter used in step (i) (and thus any other filter which is substantially identical to this filter) is a nanofilter.
There is no limitation to the feed solution used in the present invention, so long as it is substantially free from material which would foul membrane filters. The process is particularly suited to the purification of a solute from tea extract solutions. Thus it is preferred that the feed solution is a tea extract solution, preferably an aqueous tea extract solution.
The target solute can be any molecule, ion or atom which is capable of at least partly passing through a membrane filter. Preferably the solute is a low molecular weight (i.e. less than 1 kDa) bioactive compound. More preferably the target solute is an amino acid or polyphenol. In a most preferred embodiment the target solute is theanine.
The present invention also provides an apparatus suitable for performing the process of the invention.

In one embodiment the apparatus comprises:

a) a first membrane filter arranged to separate the feed solution into a first permeate and first retentate, wherein the first permeate is enriched in the target solute with respect to the feed solution;

b) a feeder means for supplying the feed solution to the first membrane filter;

c) a second membrane filter arranged to separate the first permeate into a second permeate and a second retentate, wherein the second permeate is enriched in the target solute with respect to the first permeate; and

d) a supply means for supplying at least a portion of the first permeate to the second membrane filter;

wherein the second membrane filter is substantially identical to the first membrane filter.

Further enrichment of the second permeate can be achieved by passing the second permeate through the same, or at least a substantially identical, membrane filter. Thus in a preferred embodiment the apparatus comprises at least one further membrane filter substantially identical to the first membrane filter and at least one further supply means for supplying at least a portion of the second permeate to the at least one further membrane filter. In a most preferred embodiment the apparatus comprises a series of further membrane filters substantially identical to the first membrane filter and a series of further supply means, with each of the further supply means supplying at least a portion of the permeate from one of the further membrane filters to another of the further membrane filters. For example, there may be a series of 2 to 5 further membrane filters, more preferably 2 to 3.

In another embodiment the apparatus comprises:

A) a membrane filter having an inlet and an outlet and being arranged to separate the feed solution into a permeate and retentate, wherein the permeate is enriched in the target solute with respect to the feed solution; and

B) a feeder means for supplying the feed solution to the input of the membrane filter;

wherein the apparatus further comprises a supply means arranged to recycle the permeate from the outlet to the inlet of the filter.

This embodiment minimises the need for pumps and extra filtration equipment.

## BRIEF DESCRIPTION OF THE DRAWINGS

Figure 1 is a schematic representation of an apparatus according to a first embodiment of the invention.

Figure 2 is a schematic representation of an apparatus according to a second embodiment of the invention.

## DETAILED DESCRIPTION

The present invention will be further described with reference to the following preferred embodiments and examples.

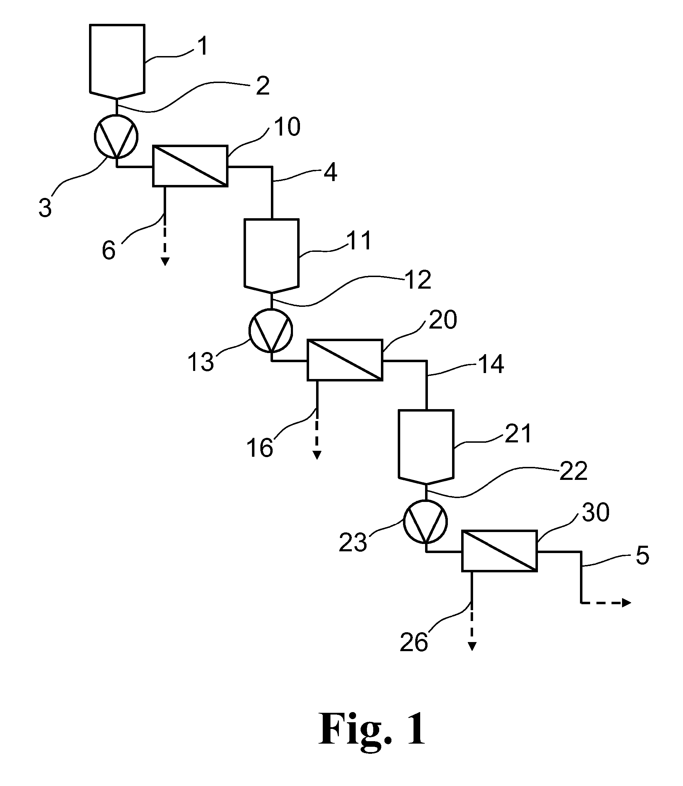

The apparatus shown in Figure 1 comprises a feed tank (1) containing the feed solution. A first conduit (2) runs from an outlet of the feed tank (1) to the inlet of a first membrane filter (10). Part-way along the first conduit (2) is a first pump (3) for pumping feed solution along the conduit (2). The first filter (10) has connected thereto a first retentate conduit (6).

An outlet of the first membrane filter (10) has connected thereto a second conduit (4) which is in fluid communication with an inlet of a first buffer tank (11). A third conduit (12) runs from an outlet of the first buffer tank (11) to the inlet of a second membrane filter (20) which is substantially identical to the first membrane filter (10). Part-way along the third conduit (12) is a second pump (13) for pumping liquid along the conduit (12).

The second membrane filter (20) has connected thereto a second retentate conduit (16).

An outlet of the second membrane filter (20) has connected thereto a fourth conduit (14) which is in fluid communication with an inlet of a second buffer tank (21). A fifth conduit (22) runs from an outlet of the second buffer tank (21) to the inlet of a third membrane filter (30) which is substantially identical to the first membrane filter (10). Part-way along the fifth conduit (22) is a third pump (23) for pumping liquid along the conduit (22). The third membrane filter (30) has connected thereto a third retentate conduit (26).

A product outlet (5) is formed by a conduit connected to the outlet of the third membrane filter (30).

In use, the first pump (3) is activated to urge feed solution from the feed tank (1) to the inlet of the first membrane filter (10) via the first conduit (2). The feed tank (1), first conduit (2) and first pump (3) thus make up a feeder means supplying the feed solution to the first membrane filter (10).

The first membrane filter (10) separates the feed solution into a first permeate and first retentate, wherein the first permeate is enriched in the target solute with respect to the feed solution. The first retentate is carried away from the first membrane filter (10) through the first retentate conduit (6).

The first permeate exits the outlet of the first membrane filter (10) and is fed, via the second conduit (4), into the first buffer tank (11). The second pump (13) is then activated to urge the first permeate solution from the first buffer tank (11) to the inlet of the second membrane filter (20) via the third conduit (12). The second conduit (4), first buffer tank (11), third conduit (12) and second pump (13) thus make up a supply means supplying the first permeate to the second membrane filter (20).

The second membrane filter (20) separates the first permeate into a second permeate and a second retentate, wherein the second permeate is enriched in the target solute with respect to the first permeate. The second retentate is carried away from the second filter (20) through the second retentate conduit (16).

The second permeate exits the outlet of the second membrane filter (20) and is fed, via the fourth conduit (14), into the second buffer tank (21). The third pump (23) is then activated to urge the second permeate solution from the second buffer tank (21) to the inlet of the third membrane filter (30) via the fifth conduit (22). The fourth conduit (14), second buffer tank (21), fifth conduit (22) and third pump (23) thus make up a supply means supplying the second permeate to the third membrane filter (30).

The third membrane filter (30) separates the second permeate into a third permeate and a third retentate, wherein the third permeate is enriched in the target solute with respect to the second permeate. The third retentate is carried away from the third membrane filter (30) through the third retentate conduit (26). The third permeate is recovered from the product outlet (5).

Although the embodiment in Figure 1 has been described with reference to three membrane filters (10, 20 and 30). It will be understood that, in addition to the first (10) and second (20) membrane filters, there may be employed a series of further membrane filters and associated supply means in place of the third membrane filter (30) in accordance with the desired end purity of the product.

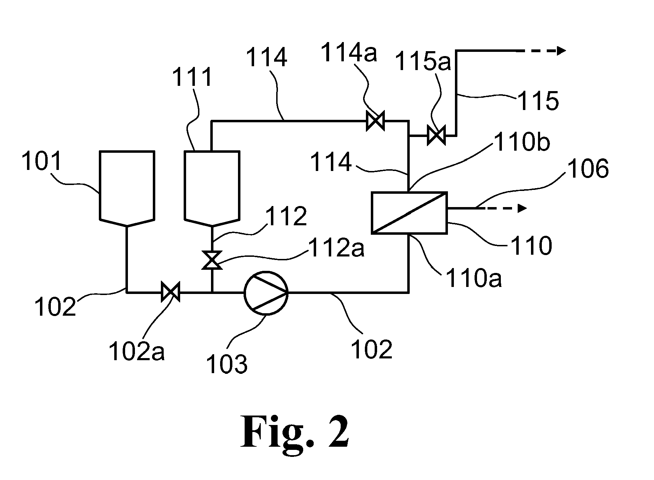

The apparatus shown in figure 2 comprises a feed tank (101) containing the feed solution. A first conduit (102) runs from an outlet of the feed tank (101) to the inlet (110a) of a membrane filter (110). Part-way along the first conduit (102) is a pump (103) for pumping feed solution along the conduit (102). A first valve (102a) is present in the first conduit (102) between the feed tank (101) and the pump (103). The membrane filter (110) has connected thereto a retentate conduit (106).

An outlet (110b) of the membrane filter (110) has connected thereto a second conduit (114) which is in fluid communication with an inlet of a buffer tank (111). Part-way along the second conduit (114) is a junction with a third conduit (115), the third conduit (115) forming a product outlet. A second valve (114a) is present in the second conduit (114) between the junction with the third conduit (115) and the buffer tank (111). A third valve (115a) is present in the third conduit (115).

A fourth conduit (112) runs from an outlet of the buffer tank (111) to a junction with the first conduit (102). The junction with the first conduit (102) is at a point between the first valve (102a) and the pump (103). A fourth valve (112a) is present in the fourth conduit (112).

In use, to begin with the first (102a) and second (114a) valves are open whilst the third (115a) and fourth (112a) valves are closed. In this configuration, the pump (103) is activated to urge feed solution from the feed tank (101) to the inlet (110a) of the membrane filter (110) via the first conduit (102). The feed tank (101), first conduit (102) and pump (103) thus make up a feeder means supplying the feed solution to the input (110a) of the membrane filter (110).

The membrane filter (110) separates the feed solution into a permeate and retentate, wherein the permeate is enriched in the target solute with respect to the feed solution. The retentate is carried away from the membrane filter (110) through the retentate conduit (106). The permeate exits the outlet (110b) of the membrane filter (110) and is fed, via the second conduit (114), into the buffer tank (111).

The first (102a) and second (114a) valves are then closed whilst the third (115a) and fourth (112a) valves are opened. The pump (103) is then activated to urge the permeate solution from the buffer tank (111) to the inlet (110a) of membrane filter (110) via the third (112) and first (102) conduits. The second conduit (114), buffer tank (111), first conduit (102), third conduit (112) and pump (103) thus make up a supply means arranged to recycle the permeate from the outlet (110b) to the inlet (110a) of the filter (110).

The membrane filter (110) separates the permeate into a further permeate and further retentate, wherein the further permeate is enriched in the target solute with respect to the original permeate. The further retentate is carried away from the membrane filter (110) through the retentate conduit (106). The further permeate exits the outlet (110b) of the membrane filter (110) and is recovered from the product outlet (115).

Although the embodiment in Figure 2 has been described with reference to a single pass of the permeate through the membrane filter (110). It will be understood that the permeate may be recycled a plurality of times through the membrane filter (110) in accordance with the desired end purity of the product.

Furthermore, although the above embodiments have been described as operated in dead-end mode, it should be understood that any or all of the filtration steps of the process of the invention may be performed as crossflow filtration. Crossflow is especially preferred as it allows for continuous operation.

**EXAMPLE**

This example demonstrates purification of theanine from an aqueous tea extract by 4 passes through the same nanofilter.

*Preparation of tea extract:*

Broken mixed fannings (BMF) of black tea were supplied by Unilever Kenya. The BMF were extracted in water at a temperature of 4°C for 10 mins. The resulting crude extract was filtered with a 0.2 $\mu$m microfilter (Amersham Biosciences type CPF-2-G-55). The final aqueous extract had a solids content of 5.5 g/l and a theanine content of 0.0002 wt%.

*Nanofiltration:*

A NTR7450 nanofilter supplied by Nitto Denko was used. This filter had a rejection percentage for theanine of less than 5%. The aqueous tea extract was passed through the nanofilter and the resulting permeate collected. This first permeate was then passed through the nanofilter and the resulting permeate collected. This second permeate was then passed through the nanofilter and the resulting permeate collected. This third permeate was then passed through the nanofilter and the resulting permeate collected.
The operating conditions were: TMP = 5 bar and Temperature = 25°C.

*Results:*

Table 1 shows the composition of the permeate after each nanofiltration step.

TABLE 1

| Solution | Theanine Concentration (% w/w of solution) | Theanine Concentration (% w/w solids) |
|---|---|---|
| Aq. Tea Extract | 0.0002 | 2.5 |
| 1st Permeate | 0.0002 | 4.9 |
| 2nd Permeate | 0.0002 | 10.8 |
| 3rd Permeate | 0.0002 | 13.2 |
| 4th Permeate | 0.0002 | 18.9 |

The data in Table 1 illustrate that theanine enrichment of a permeate from a membrane filtration step can be achieved by passing the permeate through the same membrane filter and that multiple further passes through the same filter result in even greater enrichment.

**Claims**

1.   A process for purifying a target solute from a feed solution comprising impurities, the process comprising:

   i) passing the feed solution through a first membrane filtration step thereby to form a first retentate and a first permeate, wherein the first permeate is enriched in the target solute with respect to the feed solution; and then
   ii) passing at least a portion of the first permeate through a second membrane filtration step thereby to form a second retentate and a second permeate, wherein the second permeate is enriched in the target solute with respect to first permeate;

   **characterised in that** the membrane filter used in the second membrane filtration step (ii) is substantially identical to the membrane filter used in the first membrane filtration step (i).

2. A process according to claim 1 wherein the membrane filter used in the first filtration step (i) has a rejection percentage of the target solute of $Q_1$, the membrane filter used in the second filtration step (ii) has a rejection percentage of the target solute of $Q_2$, and the ratio $Q_1:Q_2$ is from 1.2:1 to 1:1.2.

3. A process according to claim 2 wherein the ratio $Q_1:Q_2$ is from 1.15:1 to 1:1.15.

4. A process according to claim 3 wherein the same membrane filter is used in both filtration steps (i) and (ii).

5. A process according to any one of the preceding claims wherein at least a portion of the second permeate is passed through at least one further membrane filtration step using a membrane filter substantially identical to the membrane filter used in the first membrane filtration step (i).

6. A process according to claim 5 wherein at least a portion of the second permeate is passed through a series of further membrane filtration steps, each further membrane filtration step using a membrane filter substantially identical to the membrane filter used in the first membrane filtration step (i).

7. A process according to any one of the preceding claims wherein the membrane filter used in the first membrane filtration step (i) is a nanofilter.

8. A process according to any one of the preceding claims wherein the feed solution is a tea extract solution.

9. A process according to any one of the preceding claims wherein the target solute is an amino acid or polyphenol.

10. A process according to claim 9 wherein the target solute is theanine.

11. An apparatus for purifying a target solute from a feed solution comprising impurities, the apparatus comprising:

a) a first membrane filter arranged to separate the feed solution into a first permeate and first retentate, wherein the first permeate is enriched in the target solute with respect to the feed solution;
b) a feeder means for supplying the feed solution to the first membrane filter;
c) a second membrane filter arranged to separate the first permeate into a second permeate and a second retentate, wherein the second permeate is enriched in the target solute with respect to the first permeate; and
d) a supply means for supplying at least a portion of the first permeate to the second membrane filter;

**characterised in that** the second membrane filter is substantially identical to the first membrane filter.

12. An apparatus according to claim 11 wherein the first membrane filter has a rejection percentage of the target solute of $Q_1$, the second membrane filter has a rejection percentage of the target solute of $Q_2$, and the ratio $Q_1 : Q_2$ is from 1.2:1 to 1:1.2.

13. An apparatus according to claim 12 wherein the ratio $Q_1:Q_2$ is from 1.1:1 to 1:1.1.

14. An apparatus according to any one of claims 11 to 13 wherein the apparatus comprises at least one further membrane filter substantially identical to the first membrane filter and at least one further supply means for supplying at least a portion of the second permeate to the at least one further membrane filter.

15. An apparatus according to claim 14 wherein the apparatus comprises a series of further membrane filters substantially identical to the first membrane filter and a series of further supply means, wherein each of the further supply means supplies at least a portion of the permeate from one of the further membrane filters to another of the further membrane filters.

16. An apparatus according to any one of claims 11 to 15 wherein the first membrane filter is a nanofilter.

17. An apparatus for purifying a target solute from a feed solution comprising impurities, the apparatus comprising:

A) a membrane filter having an inlet and an outlet and being arranged to separate the feed solution into a permeate and retentate, wherein the permeate is enriched in the target solute with respect to the feed solution; and

B) a feeder means for supplying the feed solution to the input of the membrane filter;

**characterised in that** the apparatus further comprises a supply means arranged to recycle the permeate from the outlet to the inlet of the filter.

18. An apparatus according to claim 17 wherein the membrane filter is a nanofilter.

**Fig. 1**

Fig. 2

**European Patent Office**

**EUROPEAN SEARCH REPORT**

Application Number

EP 07 11 0978

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | DATABASE WPI<br>Section Ch, Week 199421<br>Derwent Publications Ltd., London, GB;<br>Class B02, AN 1994-173736<br>XP002460654<br>-& JP 06 116258 A (NIKKEN FOOD KK)<br>26 April 1994 (1994-04-26)<br>* abstract *<br>----- | 8,10 | INV.<br>B01D61/02<br>A23F3/16<br>A23L1/305 |
| D,Y | WO 2006/037503 A (UNILEVER PLC [GB];<br>UNILEVER NV [NL]; LEVER HINDUSTAN LTD<br>[IN]; MAVROUD) 13 April 2006 (2006-04-13)<br>* page 6, line 10 - line 26; claim 1 *<br>----- | 8,10 | |
| Y | US 5 879 733 A (EKANAYAKE ATHULA [US] ET<br>AL) 9 March 1999 (1999-03-09)<br>* claims 1,11-15 *<br>----- | 8,10 | |
| X | US 6 383 392 B1 (BONRATH WERNER [DE] ET<br>AL) 7 May 2002 (2002-05-07)<br>* column 3, line 30 - line 34; claims 1,4<br>*<br>----- | 1,5,6,<br>11,14,15 | |
| X | WO 2005/054274 A (FRESENIUS KABI AUSTRIA<br>GMBH [AT]; BOBEK MICHAEL [AT]; GAERTNER<br>SVEN [A) 16 June 2005 (2005-06-16) | 1,4-7,9,<br>11,14-18 | TECHNICAL FIELDS<br>SEARCHED (IPC)<br><br>B01D<br>A23F<br>A23L |
| Y | * page 4, line 5 - line 13; figures 1,2 *<br>* page 5, line 19 - page 7, line 5 *<br>----- | 8,10 | |

The present search report has been drawn up for all claims

| Place of search | Date of completion of the search | Examiner |
|---|---|---|
| Munich | 30 November 2007 | Goers, Bernd |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons
........................................................................
& : member of the same patent family, corresponding
   document

EPO FORM 1503 03.82 (P04C01)

## EP 2 008 702 A1

**ANNEX TO THE EUROPEAN SEARCH REPORT
ON EUROPEAN PATENT APPLICATION NO.**　　EP 07 11 0978

This annex lists the patent family members relating to the patent documents cited in the above-mentioned European search report.
The members are as contained in the European Patent Office EDP file on
The European Patent Office is in no way liable for these particulars which are merely given for the purpose of information.

30-11-2007

| Patent document cited in search report | | Publication date | Patent family member(s) | | Publication date |
|---|---|---|---|---|---|
| JP 06116258 | A | 26-04-1994 | JP | 3256883 B2 | 18-02-2002 |
| WO 2006037503 | A | 13-04-2006 | CN | 101031314 A | 05-09-2007 |
| | | | EP | 1799239 A1 | 27-06-2007 |
| US 5879733 | A | 09-03-1999 | AT | 210384 T | 15-12-2001 |
| | | | AU | 1972997 A | 10-09-1997 |
| | | | BR | 9707675 A | 13-04-1999 |
| | | | CA | 2248295 A1 | 28-08-1997 |
| | | | CN | 1214613 A | 21-04-1999 |
| | | | DE | 69709065 D1 | 24-01-2002 |
| | | | DE | 69709065 T2 | 22-08-2002 |
| | | | EP | 0884953 A1 | 23-12-1998 |
| | | | ES | 2165585 T3 | 16-03-2002 |
| | | | JP | 3174065 B2 | 11-06-2001 |
| | | | JP | 11504224 T | 20-04-1999 |
| | | | JP | 3507433 B2 | 15-03-2004 |
| | | | JP | 2001197863 A | 24-07-2001 |
| | | | WO | 9730597 A1 | 28-08-1997 |
| | | | US | 6268009 B1 | 31-07-2001 |
| | | | US | 6063428 A | 16-05-2000 |
| US 6383392 | B1 | 07-05-2002 | BR | 0006559 A | 17-07-2001 |
| | | | CA | 2325734 A1 | 16-05-2001 |
| | | | CN | 1303853 A | 18-07-2001 |
| | | | DE | 60032999 T2 | 08-11-2007 |
| | | | JP | 3620019 B2 | 16-02-2005 |
| | | | JP | 2001190933 A | 17-07-2001 |
| | | | KR | 20010070212 A | 25-07-2001 |
| | | | TW | 228125 B | 21-02-2005 |
| WO 2005054274 | A | 16-06-2005 | AU | 2003294774 A1 | 24-06-2005 |

For more details about this annex : see Official Journal of the European Patent Office, No. 12/82

12

**EP 2 008 702 A1**

**REFERENCES CITED IN THE DESCRIPTION**

**Patent documents cited in the description**

- EP 2005012464 W

- EP 2005010376 W